(19) Europäisches Patentamt
European Patent Office
Office européen des brevets

(11) Publication number: **0 257 285 A2**

(12) **EUROPEAN PATENT APPLICATION**

(21) Application number: **87110342.0**

(51) Int. Cl.[4]: **A61K 31/135**

(22) Date of filing: **17.07.87**

(30) Priority: **28.07.86 US 891200**

(43) Date of publication of application:
**02.03.88 Bulletin 88/09**

(84) Designated Contracting States:
**AT BE CH DE ES FR GB GR IT LI LU NL SE**

(71) Applicant: **NELSON RESEARCH & DEVELOPMENT COMPANY**
**1001 Health Sciences Road West**
**Irvine, CA 92715(US)**

(72) Inventor: **Horn, Alan S.**
**Noordwijkweg 3A**
**NL-9804 RA Noordhorn (GR)(NL)**

(74) Representative: **Patentanwälte Kohler - Schwindling - Späth**
**Hohentwielstrasse 41**
**D-7000 Stuttgart 1(DE)**

(54) **Method and compositions for reducing the intraocular pressure of mammals.**

(57) This invention provides a method for reducing the intraocular pressure in mammals which comprises administering an effective amount of a compound selected from the group consisting of compounds represented by the general formula:

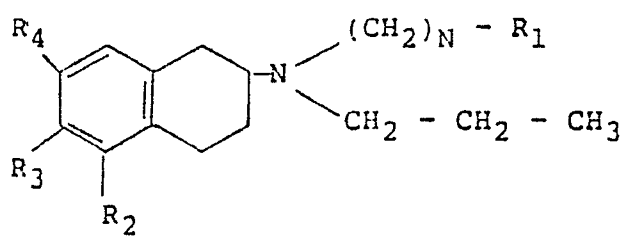

where $R_2$, $R_3$ and $R_4$ are each selected from the group consisting of H and OA; A is H or is selected from the group consisting of hydrocarbyl radicals, e.g. lower alkyl radicals, i.e. methyl, ethyl, propyl etc., or $-\underset{\underset{O}{\|}}{C}-R_5$,

wherein $R_5$ is selected from the group consisting of hydrocarbyl radicals, e.g. alkyl and aromatic residues; n is 2 or 3; and $R_1$ is selected from the group consisting phenyl and substituted phenyl, e.g. phenyl substituted with hydroxy, nitro, azido, sulfate, sulfonamido, halogen, hydrocarbyl and hetero atom-substituted hydrocarbyl radicals, wherein said heteroatoms are selected from the group consisting of halogen, nitrogen, oxygen, sulfur and phosphorus and said hydrocarbyl radicals comprise from 1 to 12 carbon atoms, with the proviso that at least one of $R_2$, $R_3$ and $R_4$ is H, that at least one of $R_2$, $R_3$ and R is not H and that $R_2$ and $R_4$ are not both OA; and pharmaceutically-acceptable salts thereof, in an ophthalmic carrier. Preferably, R is phenyl or 3 or 4 hydroxyphenyl.

EP 0 257 285 A2

# METHOD AND COMPOSITIONS FOR REDUCING THE INTRAOCULAR PRESSURE OF MAMMALS

## CROSS REFERENCE TO RELATED APPLICATIONS

This patent application is a continuation-in-part of U.S. Patent Application Serial Nos. 811,768, filed on December 20, 1985, which is a continuation-in-part of U.S Patent Application Serial No. 640,685, filed on August 13, 1984, now U.S. Patent Number 4,564,628, which is a continuation-in-part of U.S. Patent Application Serial No. 455,144, filed on January 3, 1983, and now abndoned. This patent application is also a continuation-in-part of U.S. patent Application Serial No. 839,976, filed on March 17, 1986, which is a continuation-in-part of U.S. Patent Application Serial No. 640,685, filed on August 13, 1984, now U.S. Patent No. 4,564,628, which is a continuation-in-part of U.S. Patent Application Serial No. 455,144, filed January 3, 1983 and now abandoned.. This patent application is also related to U.S. Patent Application Serial No. 455,197, filed on January 3, 1982 and now U.S. Patent No. 4,465,692.

## BACKGROUND OF THE INVENTION

### 1. Field of the Invention

This invention relates to methods of treating mammals to lower intraocular pressure and thus is useful in alleviating glaucoma. This invention also provides compositions and compounds useful in such method.

### 2. Background of the Art

Glaucoma is a disorder characaterized by increased intraocular pressure that may cause impaired vision, ranging from slight loss to absolute blindness. It has been shown that certain compounds can lower introcular pressure in various mammals. For example, it has been suggested that Bromocriptine may lower intraocular pressure in man. (See The Lancet, February 4, 1984, "Bromocriptine Eyedrops Lower Intraocular Pressure without Affecting Prolactin Levels.", by Mekki, et. al. at pages 287-288.)

Similarly, Bromocriptine, as well as lergotrile and pergolide has been shown to lower the intraocular pressure of rabbits and the latter two compounds also lowered the intraocular pressure of monkeys. (See Potter, D.E. and Burke, J. A. (1982/1983). Effects of Ergoline Derivatives on Intraocular Pressure and Iris Function in Rabbits and Monkeys. Curr. Eye Res. 2, 281-288 and Potter, D.E., Burke, J.A. and Chang, F.W. (1984). Ocular Hypotensive Action of Ergoline Derivatives in Rabbits: Effects of Sympathectomy and Domperidone Pretreatment. Curr. Eye Res. 3, 307-314.)

It has also been shown that certain dopamine analogs of the phenylethylamine class, i.e. N-methyldopamine, N,N-dimethyldopamine and N,N-di-n-propyldopamine, may alter ocular function by operating through a variety of mechanisms. However, N-methyl dopamine appeared to function by suppressing aqueous humor formation. (see Potter, D. E., Burke, J. A. and Chang, F. W. (1984). Alteration in Ocular Function Induced by Phenylethylamine Analogs of Dopamine. Curr. Eye Res. 3, 851-859.)

Finally, certain aminotetralins were shown to lower intraocular presure in rabbits. (See Burke, J. A., Chang, F. W. and Potter, D. E. (1984), Effects of Aminotetralins on Intraocular Pressure and Pupillary Function in Rabbits. J. Auton. Pharmacol. 4, 185-192.)

Thus, it is clear that many compounds have been suggested for lowering the intraocular pressure in mammals and research continues to find even more potent compounds for the treatment of glaucoma.

## SUMMARY OF THE INVENTION

This invention provides compositions useful in lowering the intraocular pressure of mammals, e.g. humans, which comprise a intraocular pressure-lowering amount of a compound selected from the group of compounds represented by the general formula:

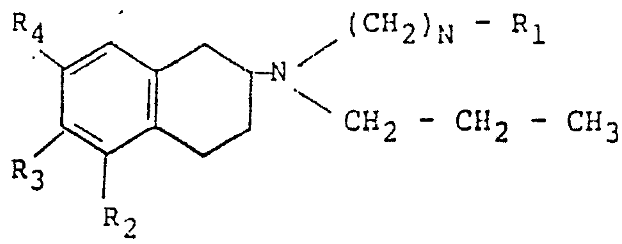

where $R_2$, $R_3$ and $R_4$ are each selected from the group consisting of H and OA; A is H or is selected from the group consisting of hydrocarbyl radicals, e.g. lower alkyl radicals, i.e. methyl, ethyl, propyl etc., or $-\underset{O}{\underset{\|}{C}}-R_5$,

wherein $R_5$ is selected from the group consisting of hydrocarbyl radicals, e.g. alkyl and aromatic residues; n is 2 or 3; and $R_1$ is selected from the group consisting of phenyl and substituted phenyl, e.g. phenyl substituted with hydroxy, nitro, azido, sulfate, sulfonamido, halogen, hydrocarbyl and hetero atom-substituted hydrocarbyl radicals, wherein said heteroatoms are selected from the group consisiting of halogen, nitrogen, oxygen, sulfur and phosphorus and said hydrocarbyl radicals comprise from 1 to 12 carbon atoms, with the proviso that at least one of $R_2$, $R_3$ and $R_4$ is H, that at least one of $R_2$, $R_3$ and $R_4$ is not H and that $R_2$ and $R_4$ are not both OA; and pharmaceutically-acceptable salts thereof in an ophthalmic carrier. Preferably, $R_1$ is phenyl or 3 or 4 hydroxyphenyl. This invention further provides a method for reducing the intraocular pressure in mammals which comprises administering an effective amount of a compound selected from the group consisting of compounds represented by the general formula:

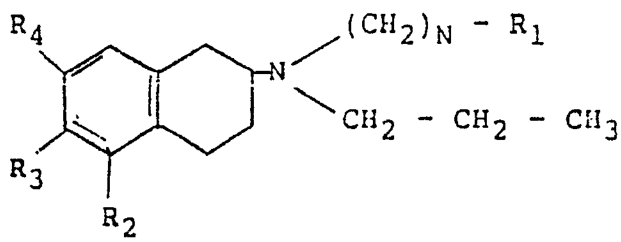

where $R_2$, $R_3$ and $R_4$ are each selected from the group consisting of H and OA; A is H or is selected from the group consisting of hydrocarbyl radicals, e.g. lower alkyl radicals, i.e. methyl, ethyl, propyl etc., or $-\underset{O}{\underset{\|}{C}}-R_5$,

wherein $R_5$ is selected from the group consisting of hydrocarbyl radicals, e.g. alkyl and aromatic residues; is 2 or 3; and $R_1$ is selected from the group consisting phenyl and substituted phenyl, e.g. phenyl substituted with hydroxy, nitro, azido, sulfate, sulfonamido, halogen,, hydrocarbyl and hetero atom-substituted hydrocarbyl radicals, wherein said heteroatoms are selected from the group consisiting of halogen,, nitrogen, oxygen, sulfur and phosphorus and said hydrocarbyl radicals comprise from 1 to 12 carbon atoms, with the proviso that at least one of $R_2$, $R_3$ and $R_4$ is H, that at least one of $R_2$, $R_3$ and R is not H and that $R_2$ and $R_4$ are not both OA; and pharmaceutically-acceptable salts thereof and an ophthalmic carrier. Preferably, R is phenyl or 3 or 4 hydroxyphenyl or 3 or 4 nitrophenyl.

## DETAILED DESCRIPTION OF THE INVENTION

The above compounds may be made by any of the methods disclosed in the U.S. patent applications and patents cited above, which have been incorporated herein by reference.

In a preferred embodiment of the instant invention $R_4$ is H and $R_2$ and $R_3$ are OA or $R_2$ is H and $R_3$ and $R_4$ are OA. That is, the catechol derivatives are one class of preferred compounds for the method and compositions of the present invention. In another preferred embodiment $R_3$ and $R_4$ are H and $R_2$ is OA or $R_2$ and $R_3$ are H and $R_4$ is OA. Preferably, A is H or $-\underset{O}{\underset{\|}{C}}-R_5$

and more preferably $R_5$ is a lower alkyl radical.

Preferably, $R_1$ is represented by the formula

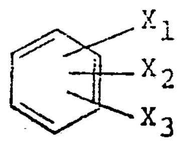

wherein each of $X_1$, $X_2$ and $X_3$ are, independently, hydrogen, hydroxy, alkoxy, nitro, cyano, azido, amino, trifluoromethyl, alkylamino, dialkylamino, halo, carboxyl, carbalkoxy, alkyl, alkenyl, alkynyl, cycloalkyl, aryl, acylamino, carboxamido, sulfonamido, and $SO_m$-(lower)alkyl, wherein m is 0, 1 or 2. More preferably, each X comprises no more than 5 carbon atoms and most preferably $X_2$ are halogen.

Even more preferably the method of the present invention comprises administering 2-(N-propyl-N-2-phenylethylamino)-5-hydroxytetralin to the eye of a mammal to reduce intraocular pressure. Moreover, the levo (-) isomer of this compound is believed to be the more active isomer for use in the method of the present invention.

Suitable ophthalmic carriers are known to those skilled in the art and all such conventional carriers may be employed in the present invention. Thus, a particular carrier may take the form of a sterile ophthalmic ointment, cream, gel, solution, or dispersion and preferably a solution. Also including as suitable ophthalmic carriers are slow releasing polymers, e.g. "Ocusert" polymers, "Hydron" polymers, etc. Stabilizers may also be used such as, for example, chelating agents, e.g. EDTA. Antioxidants may also be used, e.g. sodium bisulfite, sodium thiosulfite, 8-hydroxy quinoline or ascorbic acid. Sterility typically will be maintained by conventional ophthalmic preservatives, e.g. chlorbutanol, benzalkonium chloride, cetylpyridinium chloride, phenyl mercuric salts, thimerosal, phenethyl alcohol, etc., for aqueous formualtions, and used in amounts which are non-toxic and which generally vary from about 0.001 to about 0.1% by weight of the aqueous solution. Conventional preservatives for ointments include methyl and propyl parabens. Typical ointment bases include white petrolatum and mineral oil or liquid petrolatum. However, preserved aqueous carriers are preferred. Solutions may be manually delivered to the eye in suitable dosage form, e.g., eye drops, or delivered by suitable microdrop or spray apparatus typically affording a metered dose of medicament. Examples of suitable ophthalmic carriers or stabilizers include sterile, substantially isotonic, aqueous solutions containing minor amounts, i.e., less than about 5% by weight hydroxypropylmethylcellulose, polyvinyl alcohol, carboxymethylcellulose, hydroxyethylcellulose, glycerine, EDTA, sodium busulfite and ascorbic acid.

The amount of active compound to be used in the therapeutic treatment of glaucoma will vary with the age of the patient and the severity of the glaucoma. Generally, a dose level of one or two drops of the foregoing aqueous solution 1-4 times daily would be a suitable dosage amount. Generally, the concentration of active compound will vary between aabout 0.001 and about 5% and preferably between about 0.05 and about 1% (wt./v calculated on the basis of the free base) of said ophthalmic composition.

Preferably, the ophthalmic composition of this invention should have a pH within the range of about 4.0 to 9.0 when intended for topical application. Above and below this pH range the solution may irritate and sting the eye of the user. The solutions of the present invention may be maintained between about pH 4.0 and 7.5 with suitable amounts of buffering agents including borate, carbonate, phosphate. Tris (hydroxymethyl aminomethane), acetate and citrate buffers.

A preferred ophthalmic composition is a preserved aaqueous solution containing the following ingredients at approximately the indicated concentration.

| | |
|---|---|
| Active compound | 0.001 - 1 wt. % |
| Stabilizer | 0.01 - 0.1 wt. % |
| Preservative | 0.005 - 0.5 wt. % |
| Buffer (sufficient to maintain pH between about 4.0 and 7.5) | 0.1 - 0001 M |
| NaCl qs. ad. | (isotonic) |
| Water qs. ad. | 100% |

To illustrate the manner in which the invention may be carried out, the following examples are given. It is understood, however, that the example is for the purpose of illustration and the invention is not to be regarded as limited to any of the specific materials or conditions therein.

EXAMPLE

Male, albino New Zealand rabbits, and female Cebus apella monkeys are used in the example. Rabbits are used primarily to screen for undue ocular toxicity of 2-(N-propyl-N-2-phenylethylamino)-5-hydroxytetralin (active compound) before conducting experiments in monkeys.

A racemic mixture of 2-(N-propyl-N-2-phenylethylamino)-5-hydroxytetralin (active compound) is dissolved in distilled water (vehicle or carrier) on the day of the experiment. Solutions are administered in a masked manner, that is, solutions are prepared by a person that was neither involved in drug administration nor measurement of intraocular pressure (IOP) and pupil diameter (PD). The solution of the active compound is applied unilaterally with the contralateral (fellow) eye receiving vehicle only. Five monkeys are treated bilaterally with vehicle; one to two vehicle-treated monkeys are included each time a different dose of the active compound is used. Doses of active compound tested are: 0.165, 0.5 and 1.65 mg.

After taking two baseline (0 time) measurements, aliquots (50ul) of the solution of active compound and/or vehicle, only, are administered topically. Subsequently, IOP measurements are made at 0.5, 1, 2, 3, 4 and 5 hours post drug. The active compound is found to effectively lower the intraocular pressure of the monkey as compared to the vehicle, alone.

While particular embodiments of the invention have been described it will be understood of course that the invention is not limited thereto since many obvious modifications can be made and it is intended to include within this invention any such modifications as will fall within the scope of the appended claims.

**Claims**

1. A method for reducing the intraocular pressure in mammals which comprises administering an effective amount of a compound selected from the group consisting of compounds represented by the general formula:

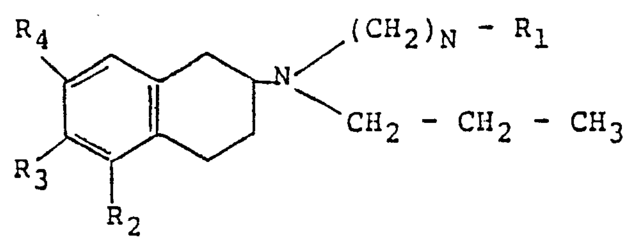

where $R_2$, $R_3$ and $R_4$ are each selected from the group consisting of H and OA; A is H or is selected from the group consisting of hydrocarbyl radicals, or $-\underset{\underset{O}{\parallel}}{C}-R_5$,

wherein $R_5$ is selected from the group consisting of hydrocarbyl radicals; n is 2 or 3; and $R_1$ is selected from the group consisting phenyl and phenyl, substituted with hydroxy, nitro, azido, sulfate, sulfonamido, halogen, hydrocarbyl and hetero atom-substituted hydrocarbyl radicals, wherein said heteroatoms are selected from the group consisting of halogen, nitrogen, oxygen, sulfur and phosphorus and said hydrocarbyl radicals commprise from 1 to 12 carbon atoms, with the proviso that at least one of $R_2$, $R_3$ and $R_4$ is H, that at least one of $R_2$, $R_3$ and R is not H and that $R_2$ and $R_4$ are not both OA; and pharmaceutically-acceptable salts thereof in an ophthalmic carrier.

2. The method of Claim 1 wherein $R_1$ is phenyl or phenyl substituted with hydroxy, nitro, sulfo, amino or methylenedioxy radicals.

3. The method of claim 2 wherein the phenyl radical comprises a single substituent having no more than 5 carbon atoms.

4. The method of Claim 1 wherein said compound is 5-hydroxy-2-(N-n-propyl-N-2-phenylethyl)-aminotetralin or a pharmaceutically-acceptable salts thereof.

5. The method of Claim 1 wherein said compound is 5-hydroxy-2-(N-n-propyl-N-2-[3-hydroxyphenyl]-ethyl)-aminotetralin or a pharmaceutically-acceptable salt thereof.

6. A composition for reducing the intraocular pressure in mammals which comprises an effective amount of a compound selected from the group consisting of compounds having the structural formula

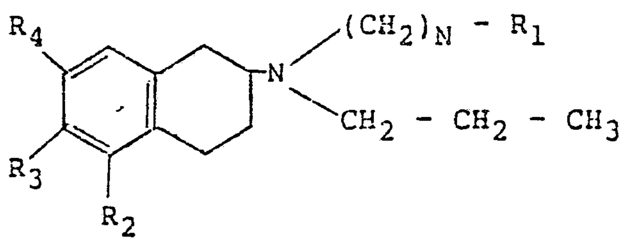

where $R_2$, $R_3$ and $R_4$ are each selected from the group consisting of H and OA; A is H or is selected from the group consisting of hydrocarbyl radicals, or is $-\underset{\underset{O}{\parallel}}{C}-R_5$,

wherein $R_5$ is selected from the group consisting of hydrocarbyl radicals, n is 2 or 3; and $R_1$ is selected from the group consisting of phenyl and substituted phenyl, phenyl substituted with hydroxy, nitro, azido, sulfate, sulfonamido, halogen hydrocarbyl and hetero atom-substituted hydrocarbyl radicals, wherein said heteroatoms are selected from the group consisting of halogen, nitrogen, oxygen, sulfur and phosphorus and said hydrocarbyl radicals comprise from 1 to 12 carbon atoms, with the proviso that at least one of $R_2$, $R_3$ and $R_4$ is H, that at least one of $R_2$, $R_3$ and R is not H and that $R_2$ and $R_4$ are not both OA; and pharmaceutically-acceptable salts thereof in an ophthalmic carrier.

7. The composition of Claim 6 wherein said ophthalmic carrier is water.

8. The composition of Claim 7, where $R_1$ is phenyl or phenyl substituted with hydroxy, alkoxy, nitro, cyano, azido, amino, trifluoromethyl, alkylamino, dialkylamino, halo, carboxyl, carbalkoxy, alkyl, alkenyl, alkynyl, cycloalkyl, aryl, acylamino, carboxamido, sulfonamido, and $SO_m$-(lower)alkyl, wherein m is 0, 1 or 2. More preferably, each comprises no more than 5 carbon atoms and most preferably $X_2$ are hydrogen.

9. The composition of claim 8 wherein the phenyl radical comprises a single substituent having more than 5 carbon atoms.

10. The composition of Claim 7 wherein said compound is 5-hydroxy-(N-n-propyl-N-2-phenylethyl)-aminotetralin and pharmaceutically-acceptable salts thereof.

11. The composition of Claim 7 wherein said compound is 5-hydroxy-2-(N-n-propyl-N-2-[3-hydroxyphenyl])-aminotetralin or a pharmaceutically-acceptable salt thereof.